# DEMANDE DE BREVET EUROPEEN

(11) **EP 4 838 219 A1**
(43) Date de publication de la demande: **11.11.2026**
(21) Numéro de dépôt: 25175176.4
(22) Date de dépôt: 08.05.2025
(51) Int. Cl.: G01N 33/497, A61B 5/00

(54) **APPAREIL ET PROCEDE DE MESURE D'ALCOOLEMIE**

(71) Demandeur: Fline, 1300 Wavre (BE)
(72) Inventeur: ROLAND, Zachary, 1300 Wavre (BE); FREH, Sabry, 1300 Wavre (BE)
(74) Mandataire: Gevers Patents

(57) **Abrégé**

Appareil (10) de mesure d'alcoolémie comprenant une enceinte (12), une entrée d'air (16) à tester accessible depuis l'extérieur de l'enceinte (12), un dispositif (14) électronique de mesure du taux d'alcool dans l'air à tester, le dispositif (14) étant dans l'enceinte (12), une chambre (18) transmettant l'air à tester depuis l'entrée d'air (16) au dispositif (14) de mesure, la chambre (18) étant dans l'enceinte (12). Procédé de mesure d'alcoolémie.

## Description

### Domaine technique

La présente invention concerne un appareil et un procédé de mesure d'alcoolémie.

### Art antérieur

Il existe des dispositifs de mesure du taux d'alcool dans l'air à tester, aussi appelés éthylotest ou éthylomètre. Ces dispositifs sont portatifs et sont utilisés pour mesurer le taux d'alcool dans l'air expiré par une personne, par exemple s'apprêtant à conduire. L'inconvénient de ces dispositifs est que leur utilisation hors surveillance peut conduire à une mauvaise exécution du test.

Il y a un besoin pour un appareil de mesure d'alcoolémie qui puisse être utilisé de manière fiable hors surveillance.

### Exposé de l'invention

À cet effet, l'invention propose un appareil de mesure d'alcoolémie comprenant : une enceinte, une entrée d'air à tester accessible depuis l'extérieur de l'enceinte, un dispositif électronique de mesure du taux d'alcool dans l'air à tester, le dispositif étant dans l'enceinte, une chambre transmettant l'air à tester depuis l'entrée d'air au dispositif de mesure, la chambre étant dans l'enceinte.

Selon une variante, l'entrée d'air est configurée pour dévier l'introduction de l'air à tester du dispositif de mesure.

Selon une variante, l'appareil comprend des organes de chauffage de la chambre.

Selon une variante, l'appareil comprend des capteurs de mesure de la température dans la chambre, les organes de chauffage étant configurés pour chauffer selon la température dans la chambre.

Selon une variante, l'appareil comprend une plaque métallique supportant les organes de chauffage, les organes de chauffage étant aptes à chauffer la plaque métallique selon la température dans la chambre.

Selon une variante, l'appareil comprend en outre une ventilation apte à aérer la chambre.

Selon une variante, la chambre comprend en outre un orifice de ventilation et un orifice d'évacuation d'air, les orifices définissant une aération de la chambre entre eux.

Selon une variante, la chambre comprend une paroi inférieure inclinée depuis l'entrée d'air, la paroi inférieure étant apte à évacuer un liquide hors de la chambre.

Selon une variante, l'appareil comprend en outre une valve anti-reflux à l'entrée d'air et/ou à un orifice de ventilation le cas échéant.

Selon une variante, la chambre a un volume supérieur à une gorgée de liquide susceptible d'être introduite par un utilisateur dans l'appareil.

Selon une variante, l'appareil comprend en outre un adaptateur configuré pour l'introduction d'air dans la chambre par l'entrée d'air, l'adaptateur étant de préférence amovible et à usage unique.

Selon une variante, l'appareil étant une borne.

L'invention se rapporte aussi à un procédé de mesure d'alcoolémie à l'aide de l'appareil tel que décrit précédemment, comprenant l'introduction d'air à tester dans la chambre par l'entrée d'air, et la mesure du taux d'alcool dans l'air à tester par le dispositif de mesure.

Selon une variante, le procédé comprend en outre l'aération de la chambre avant et/ou après l'introduction d'air à tester et la mesure du taux d'alcool.

Selon une variante, le procédé comprend en outre le chauffage de la chambre et/ou l'évacuation de liquide introduit dans l'appareil.

L'usage, dans ce document, du verbe « comprendre », de ses variantes, ainsi que ses conjugaisons, ne peut en aucune façon exclure la présence d'éléments autres que ceux mentionnés. L'usage, dans ce document, de l'article indéfini « un », « une », ou de l'article défini « le », « la » ou « l' », pour introduire un élément n'exclut pas la présence d'une pluralité de ces éléments.

L'ensemble des modes de réalisation préférés ainsi que l'ensemble des avantages de l'appareil selon l'invention se transposent mutatis mutandis au présent procédé. Les différents modes de réalisation peuvent être considérés seuls ou en combinaison.

### Brève description des figures

D'autres caractéristiques et avantages de la présente invention apparaîtront à la lecture de la description détaillée qui suit pour la compréhension de laquelle on se reportera aux figures annexées qui montrent :
- la figure 1, une vue schématique de l'appareil selon l'invention ;
- la figure 2, une vue de l'intérieur de l'appareil ;
- la figure 3, une vue de la chambre en coupe transversale ;
- la figure 4, une vue d'un détail de l'appareil ;
- la figure 5, une vue d'un détail de l'appareil.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée de modes de réalisation de l'invention

L'invention se rapporte à un appareil de mesure d'alcoolémie comprenant une enceinte, une entrée d'air à tester accessible depuis l'extérieur de l'enceinte, un dispositif électronique de mesure du taux d'alcool dans l'air à tester, le dispositif étant dans l'enceinte, et une chambre transmettant l'air à tester vers le dispositif de mesure du taux d'alcool, la chambre étant dans l'enceinte. L'appareil permet de protéger le dispositif de mesure, ce qui permet d'utiliser l'appareil de manière fiable et répétée hors surveillance. L'appareil permet de faire des mesures fiables dans des conditions variables en créant un environnement stable et sûr pour l'exécution du test d'alcoolémie.

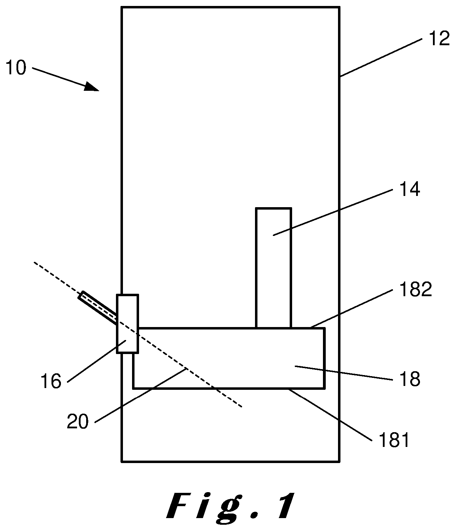

La figure 1 montre une vue schématique de l'appareil 10 de mesure d'alcoolémie selon l'invention. L'appareil 10 comprend une enceinte 12 dans laquelle se trouve un dispositif 14 de mesure du taux d'alcool dans l'air à tester. Le dispositif 14 est un éthylotest ou éthylomètre. Il s'agit par exemple d'un éthylotest ou éthylomètre de type portatif, en main, ou bien d'une carte électronique. Le dispositif 14 peut être fixé de manière détachable ou de manière intégré dans l'appareil 10. Le dispositif 14 peut être clipsé. La fixation du dispositif 14 peut être renforcée par une fixation additionnelle, par exemple de type auto agrippante. En particulier, le dispositif 14 est un dispositif électronique de mesure du taux d'alcool dans l'air à tester. Le dispositif 14 électronique est en particulier équipé d'une sonde ou de capteurs, électrochimiques par exemple, qui réagissent à la présence d'alcool dans l'air expiré. Le dispositif 14 donne une valeur chiffrée de taux d'alcoolémie (à la différence d'un éthylotest chimique montrant un changement de couleur de cristaux par exemple). Le dispositif 14 peut être calibré pour détecter l'atteinte d'un seuil de détection. Si ce seuil est atteint, le dispositif 14 détecte que le dépistage est positif (ou négatif dans la cas contraire). Le dispositif 14 peut donc délivrer une valeur chiffrée de taux l'alcoolémie et/ou l'atteinte d'un seuil de détection. Le dispositif 14 est réutilisable. Le dispositif 14 peut être réutilisé par exemple plusieurs centaines de fois. Le dispositif 14 n'est pas jetable après une utilisation. Le dispositif 14 n'est pas d'utilisation unique. Le calibrage du dispositif 14 peut être réalisé pour une longue durée, par exemple pour plusieurs mois. Le dispositif 14 est au sein de l'enceinte. Le dispositif 14 est confiné dans l'enceinte 12. Le dispositif 14 n'est pas accessible par l'utilisateur.

L'appareil 10 comprend également une entrée d'air 16 à tester. L'entrée d'air 16 est accessible depuis l'extérieur de l'enceinte 10. L'entrée d'air 16 permet l'introduction de l'air dans l'appareil 10. L'entrée d'air 16 est une interface permettant à l'utilisateur d'introduire de l'air dans l'appareil 10. L'appareil comprend en outre une chambre 18 dans l'enceinte 12. L'introduction d'air à tester n'est pas directement dans le dispositif 14 mais par l'intermédiaire de la chambre 18. La chambre 18 transmet l'air à tester depuis l'entrée d'air 16 au dispositif 14 de mesure. La chambre est configurée pour stabiliser l'air à tester. La chambre 18 est une zone tampon entre l'entrée d'air 16 et le dispositif 14 de mesure. La chambre 18 est au sein de l'enceinte 12. La chambre 18 est confinée dans l'enceinte 12. La chambre 18 n'est pas accessible par l'utilisateur. L'appareil 10 permet de protéger son contenu contre des actes malveillants de l'utilisateur. En particulier, l'appareil 10 permet de protéger le dispositif 14 et la chambre 18 contre des actes malveillants de l'utilisateur. Ceci permet de disposer l'appareil 10 en libre-service. L'appareil 10 peut être utilisé sans l'intervention de la force publique. L'appareil 10 permet de protéger le dispositif 14 contre tout fluide (crachat, vomi, alcool, etc.) provenant de l'utilisateur. Non seulement l'enceinte 10 protège le dispositif 14 mais aussi la chambre 18 protège le dispositif 14 contre tout fluide car l'air à tester parvient indirectement au dispositif 14 via la chambre 18. La chambre 18 peut collecter tout fluide projeté par l'utilisateur pour éviter que le fluide parvienne au dispositif 14.

En particulier, l'entrée d'air 16 est configurée pour dévier l'introduction de l'air à tester du dispositif 14 de mesure. L'entrée d'air 16 permet de diriger l'introduction de l'air à tester selon une direction 20 dans la chambre 18 qui ne rencontre pas le dispositif 14 de mesure. La direction 20 de d'air introduit dans l'appareil 10, et en particulier dans la chambre 18, n'est pas sécante avec le dispositif 14. L'air est introduit dans la chambre dans une direction autre que vers le dispositif 14 puis l'air se propage dans la chambre 18 pour parvenir au dispositif 14 de mesure. Cela augmente encore la protection du dispositif 14 de mesure contre toute projection de fluide accompagnant l'air à tester. Par exemple, la direction 20 est telle que l'air à tester est dirigé vers une paroi de la chambre 18 autre qu'une paroi supportant le dispositif 14. Notamment, l'air à tester peut être dirigé vers une paroi inférieure 181 de la chambre alors que le dispositif 14 peut être positionné sur une paroi supérieure 182 de la chambre. L'air à tester se diffuse ensuite dans la chambre 18 pour atteindre le dispositif 14 de mesure.

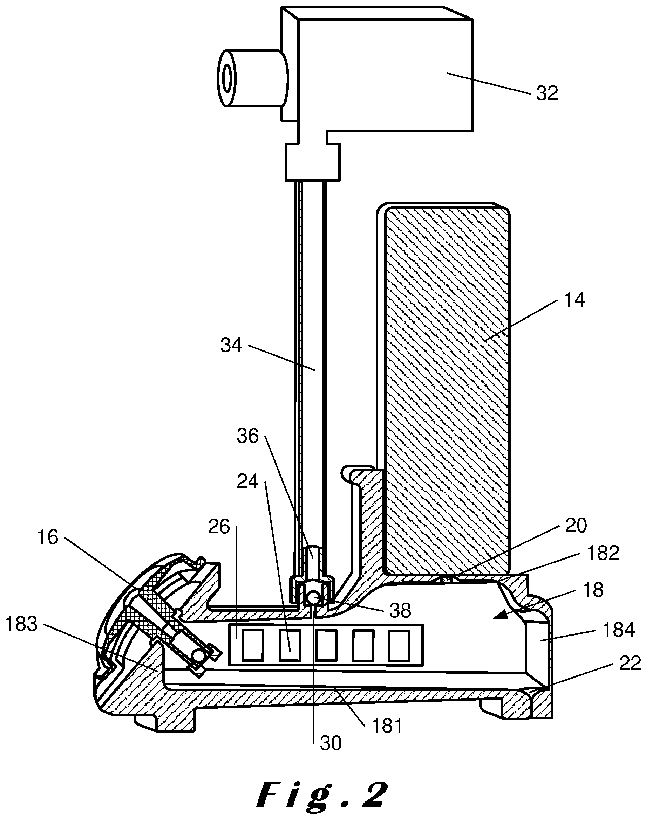

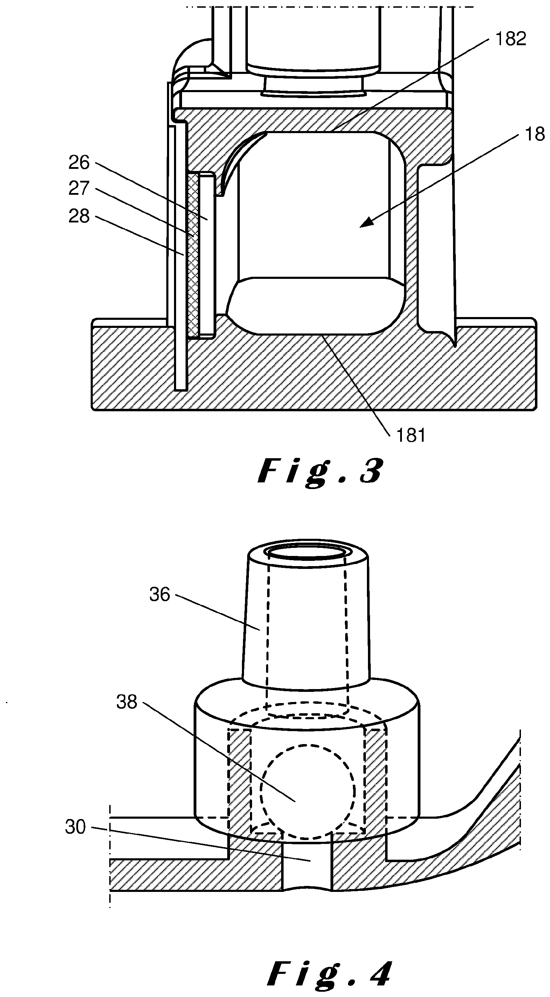

La figure 2 montre une vue de l'intérieur de l'appareil 10. L'enceinte 10 n'est pas représentée. La chambre 18 permet de transmettre l'air à tester depuis l'entrée d'air 16 au dispositif 14 de mesure. La chambre 18 favorise la distribution aérodynamique du flux d'air à tester depuis l'entrée d'air 16 au dispositif 14 de mesure. La forme géométrique de la chambre n'a pas d'incidence sur la technologie développée. La figure 2 montre que la direction d'introduction de l'air à tester par l'entrée d'air 16 dans la chambre 18 n'est pas sécante avec le dispositif 14 de mesure. La chambre 18 a une forme allongée avec des parois inférieure 181, supérieure 182 reliées par des parois d'extrémités 183, 184. La chambre 18 a une forme allongée, étendue. La figure 3 montre une vue de la chambre 18 en coupe transversale. La chambre 18 a une forme permettant une bonne transmission de l'air depuis l'entrée d'air 16 jusqu'au dispositif 14 de mesure. La chambre 18 peut avoir une section circulaire (pour avoir un volume cylindrique). Les parois supérieure, inférieure et latérales peuvent être aplaties. La chambre 18 peut avoir une forme de tube aplati. La chambre 18 peut avoir un volume supérieur à une gorgée de liquide introduite par l'utilisateur dans l'appareil 10. Par exemple, la chambre 18 peut avoir un volume d'environ 100ml. La section transversale de la chambre peut présenter une longueur entre 150mm et 200mm, une largeur entre 30mm et 40mm et une hauteur entre 25mm et 35mm (à titre d'exemple). La chambre 18 n'est pas comparable à un espace entre un cône de soufflage et une sonde d'un éthylotest portatif - au moins en ce que le volume de la chambre 18 est plus grand.

Le dispositif 14 de mesure peut être fixé à la chambre 18, par exemple sur la paroi supérieure 182 de la chambre 18. Le dispositif 14 est fixé à distance de l'entrée d'air 16. Ceci permet encore de protéger le dispositif 14 de mesure. Un passage 20 met la chambre 18 en communication (fluidique) avec le dispositif 14 de mesure afin de faire passer l'air à tester depuis la chambre 18 vers le dispositif 14 de mesure.

Comme cela est visible sur la figure 2, la paroi inférieure 181 de la chambre 18 peut être inclinée depuis la paroi d'extrémité 183 de la chambre 18 occupée par l'entrée d'air 16 vers la paroi d'extrémité 184 opposée de la chambre 18. Cette paroi inclinée donne une forme conique à la chambre 18. Ceci permet l'écoulement de tout liquide (crachat, vomi, alcool, etc...) introduit dans la chambre 18 et l'évacuation hors de la chambre 18. La chambre 18 assure une zone tampon, notamment de récolte de liquide étranger au test de taux d'alcool. Une évacuation 22 peut être prévue pour vider la chambre 18. L'évacuation est de préférence au bas de la paroi inférieure 181 inclinée, au pied de la paroi d'extrémité 184.

Selon la figure 2, l'appareil 10 peut comprendre des organes de chauffage 24 de la chambre 18. Ceci permet de maintenir l'ambiance de la chambre 18 à une température du corps (37°C). Ceci permet d'éviter la formation d'une condensation (pouvant contenir des molécules d'alcool) de l'air à tester introduit au contact des parois de la chambre. L'appareil 10 pouvant être disposé en tout lieu (fermé ou à l'air libre), les organes de chauffage 24 permettent de garantir une température correcte de la chambre 18. La chambre 18 est une zone de chauffage. Alors même que le dispositif 14 de mesure est éloigné de la bouche de l'utilisateur, les conditions de températures dans la chambre ne changent pas par rapport à l'introduction de l'air à tester directement dans un éthylotest portatif. L'air à tester introduit dans la chambre 18 reste donc à la température corporelle le temps d'être testé par le dispositif 14 de mesure. Ainsi, on évite de fausser les mesure du taux d'alcool dans l'air à tester. Les organes de chauffage 24 permettent de chauffer l'intérieur de la chambre 18. Les organes de chauffage peuvent être dans la chambre 18 (selon les figures 2 ou 3) ou pas. Les organes de chauffage 24 peuvent filaires ou sous forme de résistances. Le nombre d'organes de chauffage 24 est adapté selon leur puissance et la taille de la chambre 18.

L'appareil 10 peut comprendre des capteurs de mesure de la température dans la chambre 18 (les capteurs ne sont pas visibles sur les figures). Les organes de chauffage 24 sont configurés pour chauffer selon la température dans la chambre. Ainsi, selon le moment de l'utilisation et l'emplacement de l'appareil, la température de la chambre 18 peut varier. Les capteurs permettent d'adapter la puissance de chauffage. Ceci permet de calibrer au mieux la température dans la chambre 18.

Par ailleurs, les organes de chauffage 24 peuvent être supportés par une plaque métallique 26. Les organes de chauffage 24 sont aptes à chauffer la plaque métallique 26 selon la température dans la chambre. La plaque métallique 26 permet une bonne diffusion de la chaleur dans la chambre 18. La plaque métallique 26 peut être une paroi de la chambre 18. A titre d'exemple sur la figure 3, une paroi latérale de la chambre est formée par la plaque métallique 26. Une face de la plaque métallique 26 portant les organes de chauffage 24 est vers l'intérieur de la chambre. Un capot 28 est ajouté sur l'autre face de la plaque métallique 26 afin d'assurer une fermeture hermétique de la chambre 18. De plus, entre la plaque métallique 26 et le capot 28, il est envisageable d'intercaler un élément supplémentaire, ou tampon 27. Le tampon 27 peut être un matériau souple ou dur qui fournit une isolation électronique tout en assurant un bon transfert thermique. Le tampon 27 peut être par exemple une feuille entre 0,5mm et 2mm d'épaisseur, par exemple 1mm d'épaisseur. Le tampon 27 peut être semblable à de la pâte adhésive qui permet d'isoler les composants électroniques du métal. Ceci permet d'éviter un court-circuit tout en permettant de transférer la chaleur à la plaque métallique et donc à la chambre.

De manière avantageuse, l'appareil 10 peut comprendre en outre une ventilation apte à aérer la chambre 18. La chambre est une zone d'aération (ou ventilation). La ventilation permet une aération de la chambre et donc le renouvellement de l'air dans la chambre 18. L'aération peut avoir lieu entre deux tests (d'un même utilisateur ou de deux utilisateurs différents). L'aération peut avoir lieu avant et après un test, ce qui améliore la qualité de l'air dans la chambre 18. Ceci permet d'éviter de fausser les tests de mesure du taux d'alcool. La ventilation peut être calibrée. La figure 2 montre un exemple de ventilation. La ventilation peut comprendre un orifice de ventilation 30 permettant l'injection d'air frais dans la chambre 18 et un orifice d'évacuation de l'air injecté après aération. L'orifice d'évacuation peut être l'évacuation 22 ou bien tout autre orifice. L'orifice de ventilation 30 et l'orifice d'évacuation définissent une aération de la chambre 18 entre eux. Les orifices sont de préférence tels que l'air frais traverse la chambre 18 pour assurer une bonne aération de la chambre 18. L'orifice de ventilation 30 est par exemple sur la paroi supérieure 182. L'orifice de ventilation 30 peut être entre l'entrée d'air 16 et le dispositif 14 de mesure ce qui permet une aération en amont du dispositif 14. L'orifice d'évacuation, tel que l'évacuation 22, est de préférence à distance de l'orifice de ventilation 30. L'orifice d"évacuation peut être sur la paroi inférieure 181.

La circulation d'air dans la chambre 18 pour en assurer l'aération peut avoir lieu par simple courant d'air entre les orifices. La ventilation peut comprendre de préférence un ventilateur 32 favorisant l'injection d'air dans la chambre. Le ventilateur 32 est un organe permettant de forcer la circulation d'air dans la chambre 18 entre les orifices, ce qui améliore l'aération. Le ventilateur 32 peut être une pompe d'aspiration d'air ou un souffleur (« blower » en anglais). Le ventilateur 32 aspire de l'air frais (de préférence depuis l'extérieur de l'enceinte 12) et l'injecte dans la chambre 18. Un conduit 34 permet de déporter le ventilateur 32 à distance de l'entrée d'air 16, par exemple, pour être à distance de l'utilisateur. Le ventilateur 32 peut aspirer de l'air frais depuis le sommet de l'enceinte 12 ou par le dessous de l'enceinte.

La figure 4 montre un détail de l'appareil 10. En particulier, la figure 4 montre la connexion entre le conduit 34 et l'orifice de ventilation 30. La connexion peut être faite par un connecteur 36. Le connecteur 36 assure l'étanchéité entre le conduit 34 et l'orifice de ventilation 30. Le connecteur 36 peut comprendre une valve anti-reflux 38. La valve anti-reflux 38 permet l'injection d'air dans la chambre 18 par l'orifice de ventilation 30 pour l'aération mais empêche l'évacuation d'air par l'orifice de ventilation 30 (au profit de l'orifice d'évacuation). La valve anti-reflux 38 est par exemple une bille laissant passer l'air vers la chambre 18 mais empêchant le passage de tout air en sortie de la chambre 18. Pour cela, l'orifice de ventilation 30 peut avoir une forme de croix, la bille étant, au repos, sur la croix. Les branches de la croix s'étendent au-delà de la bille, permettant de laisser passer l'air depuis le conduit 34 vers la chambre 18. Pour empêcher un reflux d'air en sortie de la chambre 18, la bille est plaquée (vers le haut sur la figure 4) contre un canal de circulation d'air du connecteur 36 coopérant avec le conduit 34. Le canal de circulation d'air du connecteur 36 est alors obturé, empêchant l'air de remonter vers le conduit 34.

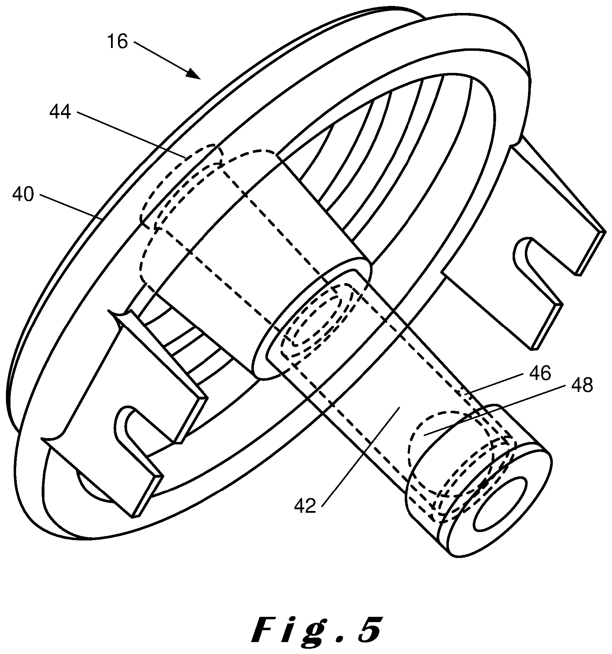

La figure 5 montre un détail de l'appareil 10. En particulier la figure 5 montre une exemple de l'entrée d'air 16. L'entrée d'air 16 assure l'interface entre l'extérieur de l'enceinte 12 (et de la chambre 18) et l'intérieur de la chambre 18. L'entrée d'air 16 est accessible par l'utilisateur. L'entrée d'air 16 est la seule partie visible par l'utilisateur (la chambre 18 et le dispositif 14 étant à l'intérieur de l'enceinte 12, donc ni accessibles ni visibles par l'utilisateur). L'entrée d'air 16 peut comprendre un disque 40 connecté à un canal 42. Le disque 40 est à l'extérieur de l'enceinte 12 et le canal 42 relie le disque 40 à l'intérieur de la chambre 18. Le canal 42 s'étend depuis une embouchure 44 au travers du disque 40 à l'intérieur de la chambre 18 pour permettre l'introduction d'air à tester dans la chambre 18. L'utilisateur peut souffler à même l'entrée d'air 16 (par l'embouchure 44). De préférence, l'appareil 10 comprend un adaptateur configuré pour l'introduction d'air dans la chambre 18 par l'entrée d'air 16, l'adaptateur étant de préférence amovible et/ou à usage unique. L'adaptateur est préféré pour des raisons sanitaires. L'adaptateur peut être un adaptateur buccal, tel qu'un embout buccal, un cône de soufflage ou encore une paille que l'utilisateur insère dans l'embouchure 44 de l'entrée d'air 16 le temps de l'introduction de l'air. L'adaptateur peut être d'utilisation unique. L'appareil 10 peut mettre des adaptateurs à disposition de l'utilisateur.

L'entrée d'air 16 peut aussi comprendre une valve anti-reflux 48. La valve anti-reflux 48 permet l'introduction d'air à tester dans la chambre 18 par l'entrée d'air 16 mais empêche l'évacuation d'air par l'entrée d'air 16 (au profit de la transmission de l'air à tester vers le dispositif 14 de mesure). La valve anti-reflux 48 évite à l'utilisateur d'aspirer de l'air « contaminé » par un utilisateur précédent, s'il est tenté d'aspirer. La valve anti-reflux 48 est par exemple une bille laissant passer l'air à tester vers la chambre 18 mais empêchant le passage de tout air en sortie de la chambre 18. Pour cela, une ouverture 46 dans la paroi du canal 42 permet de laisser passer l'air par l'entrée d'air 16 vers la chambre 18. Pour empêcher un reflux d'air en sortie de la chambre 18, la bille est plaquée en direction du disque 40 (vers le haut à gauche sur la figure 5). Le canal 42 est alors obturé, empêchant l'air de remonter depuis la chambre 18 vers l'embouchure 44.

L'entrée d'air 16 peut en outre être illuminée de manière à guider l'utilisateur lors de l'utilisation de l'appareil 10. Par exemple, l'entrée d'air 16 peut être illuminée d'une certaine couleur ou manière pour attirer l'attention de l'utilisateur vers cette entrée d'air. Pendant l'introduction d'air à tester, l'entrée d'air 16 peut rester illuminée différemment, invitant l'utilisateur à continuer à souffler. L'entrée d'air 16 peut être illuminée d'une autre manière encore pendant la mesure du taux par le dispositif 14 de mesure. Une certaine illumination peut être prévue pour afficher le résultat, pour indiquer que le seuil n'est pas atteint et une autre illumination encore pour indiquer que le seuil est dépassé. Une carte électronique portant les organes d'illumination tels que des LEDs peut être prévue.

L'appareil 10 peut par exemple être disposé dans tout type de lieux susceptibles d'accueillir des personnes sous influence de l'alcool (tels que, mais pas uniquement, des bars ou dans une salle de concert). L'appareil 10 peut être utilisé dans des situations chaotiques (utilisateurs alcoolisés) et sans surveillance - à la différence d'un éthylotest portatif. L'appareil 10 peut être une borne. La borne peut être installée à demeure (la borne est par exemple fixée au sol ou à un mur). La borne peut aussi être installée de manière provisoire sur un site (par exemple provisoirement sur le site d'un festival à durée limitée). L'appareil 10 peut être alimenté en courant par le secteur.

L'appareil 10 permet de créer un environnement stable pour l'utilisation du dispositif 14 de mesure. L'appareil 10 assure de manière fiable et répétitive la mesure du taux d'alcool. L'appareil 10 permet d'utiliser le dispositif 14 de mesure dans un environnement stable. L'appareil est en particulier configuré pour une utilisation sans surveillance (de la force publique par exemple). La chambre 18 assure une ou plusieurs fonctions de zone tampon, de zone de chauffage et/ou de zone d'aération (ou ventilation). Ces fonctions sont assurées de préférence sans séparation physique entre différentes zones de la chambre. L'appareil 10 est calibré (taille et forme de la chambre 18, température, aération, dispositif 14, etc.) de sorte à être conforme à toute directive en vigueur.

Une unité de contrôle non visible permet le contrôle du fonctionnement de l'appareil 10. L'unité de contrôle permet le contrôle du dispositif 14 et de transmettre à l'utilisateur le résultat du dispositif 14. L'unité de contrôle peut assurer la comparaison du taux mesuré par rapport à des seuils pour conduire ou non. L'unité de contrôle permet d'indiquer à l'utilisateur comment il peut procéder à un test. Un écran (possiblement tactile) permet de transmettre les informations à l'utilisateur (tel que les étapes pour effectuer le test, le taux d'alcool mesuré). L'écran peut être supporté par l'enceinte 12, par exemple sur une même face que celle supportant l'entrée d'air 16. L'unité de contrôle permet le contrôle du chauffage de la chambre. L'unité de contrôle permet aussi de déclencher l'aération (par la ventilation). L'unité peut aussi provoquer l'illumination de tout ou partie de l'appareil 10, par exemple de l'entrée d'air 16 comme décrit.

L'invention se rapporte aussi à un procédé de mesure d'alcoolémie à l'aide de l'appareil 10. Le procédé comprend l'introduction d'air à tester dans la chambre 18 par l'entrée d'air 16 et la mesure du taux d'alcool dans l'air à tester par le dispositif 14 de mesure. Le procédé peut comprendre en outre l'aération de la chambre avant et/ou après l'introduction d'air à tester et la mesure du taux d'alcool. Par sécurité, l'aération a lieu avant et après l'introduction de l'air à tester et la mesure du taux d'alcool. Le procédé peut aussi comprendre le chauffage de la chambre - à l'aide des organes de chauffage 24. Le chauffage peut avoir lieu de manière ponctuelle lors de l'utilisation de l'appareil 10 ou bien sur des plages prolongées de temps. Les plages sont programmables via l'unité de contrôle. En outre, le procédé peut comprendre l'évacuation de liquide introduit dans l'appareil, en particulier introduit par l'utilisateur.

Il apparaîtra de façon évidente à l'homme du métier que l'invention n'est pas limitée aux réalisations et aux exemples illustrés et/ou décrits ci-dessus, mais que sa portée est plus largement définie par les revendications ci-après introduites.

## Revendications

1. Appareil (10) de mesure d'alcoolémie comprenant :
- Une enceinte (12) ;
- Une entrée d'air (16) à tester accessible depuis l'extérieur de l'enceinte (12) ;
- Un dispositif (14) électronique de mesure du taux d'alcool dans l'air à tester, le dispositif (14) étant dans l'enceinte (12) ;
- Une chambre (18) transmettant l'air à tester depuis l'entrée d'air (16) au dispositif (14) de mesure, la chambre (18) étant dans l'enceinte (12).

2. Appareil (10) selon la revendication précédente, dans lequel l'entrée d'air (16) est configurée pour dévier l'introduction de l'air à tester du dispositif (14) de mesure.

3. Appareil (10) selon l'une des revendications précédentes, comprenant des organes de chauffage (24) de la chambre (18).

4. Appareil (10) selon la revendication précédente, comprenant des capteurs de mesure de la température dans la chambre (18), les organes de chauffage (24) étant configurés pour chauffer selon la température dans la chambre (18).

5. Appareil (10) selon la revendication précédente, comprenant une plaque métallique (26) supportant les organes de chauffage (24), les organes de chauffage étant aptes à chauffer la plaque métallique (26) selon la température dans la chambre (18).

6. Appareil (10) selon l'une des revendications précédentes, comprenant en outre une ventilation apte à aérer la chambre (18).

7. Appareil (10) selon la revendication précédente, dans lequel la chambre (18) comprend en outre un orifice de ventilation (30) et un orifice d'évacuation d'air, les orifices définissant une aération de la chambre (18) entre eux.

8. Appareil (10) selon l'une des revendications précédentes, dans lequel la chambre (18) comprend une paroi inférieure (181) inclinée depuis l'entrée d'air (16), la paroi inférieure (181) étant apte à évacuer un liquide hors de la chambre (18).

9. Appareil (10) selon l'une des revendications précédentes, comprenant en outre une valve anti-reflux (48, 38) à l'entrée d'air (16) et/ou à un orifice de ventilation (30) le cas échéant.

10. Appareil (10) selon l'une des revendications précédentes, dans lequel la chambre (18) a un volume supérieur à une gorgée de liquide susceptible d'être introduite par un utilisateur dans l'appareil (10).

11. Appareil (10) selon l'une des revendications précédentes, comprenant en outre un adaptateur configuré pour l'introduction d'air dans la chambre (18) par l'entrée d'air (16), l'adaptateur étant de préférence amovible et à usage unique.

12. Appareil (10) selon l'une des revendications précédentes, l'appareil étant une borne.

13. Procédé de mesure d'alcoolémie à l'aide de l'appareil (10) selon l'une des revendications précédentes, comprenant :
- L'introduction d'air à tester dans la chambre (18) par l'entrée d'air (16) ;
- La mesure du taux d'alcool dans l'air à tester par le dispositif (14) de mesure.

14. Procédé selon la revendication précédente, comprenant en outre l'aération de la chambre (18) avant et/ou après l'introduction d'air à tester et la mesure du taux d'alcool.

15. Procédé selon l'une des deux revendications précédentes, comprenant en outre le chauffage de la chambre (18) et/ou l'évacuation de liquide introduit dans l'appareil.
